# EUROPÄISCHE PATENTSCHRIFT

(11) **EP 2 956 763 B1**
(45) Veröffentlichungstag und Bekanntmachung des Hinweises auf die Patenterteilung: **31.07.2019**
(21) Anmeldenummer: 14711678.4
(22) Anmeldetag: 13.02.2014
(51) Int. Cl.: G01N 25/72, G01N 33/38

(54) **VERFAHREN UND VORRICHTUNG ZUR DETEKTION VON PARTIKELN IN GLAS**
METHOD AND DEVICE FOR DETECTING PARTICLES IN GLASS
PROCÉDÉ ET DISPOSITIF POUR DÉTECTER DES PARTICULES DANS DU VERRE

(30) Priorität: 15.02.2013 DE 102013002602
(43) Veröffentlichungstag der Anmeldung: 23.12.2015
(73) Patentinhaber: HEGLA boraident GmbH & Co. KG, 37688 Beverungen (DE)
(72) Erfinder: RAINER, Thomas, 38855 Wernigerode (DE); SCHNEIDER, Jens, 64285 Darmstadt (DE); HILCKEN, Jonas, 60385 Frankfurt (DE); DUBIEL, Manfred, 06198 Salzatal (DE)
(74) Vertreter: Patent- und Rechtsanwälte Dr. Solf & Zapf
(86) Internationale Anmeldenummer: PCT/EP2014/000392
(87) Internationale Veröffentlichungsnummer: WO 2014/124754

(56) Entgegenhaltungen:
- EP-A1- 0 189 551
- WO-A1-2006/074494
- WO-A1-2007/051582
- US-A- 4 564 761

## Beschreibung

Die vorliegende Erfindung betrifft ein neues, zuverlässiges Verfahren und Vorrichtung zur Detektion bevorzugt auch zur Identifizierung von insbesondere metallischen Partikeln in Glas, z.B. zum Zwecke der Qualitätssicherung bei der Herstellung und Verarbeitung von Glas. Einschlüsse, insbesondere metallische Einschlüsse entstehen beim Glasherstellungsprozeß durch Anreicherung von Verunreinigung der Glasrohstoffe. Die Einschlüsse besitzen eine Größe von einigen Mikrometern bis in den Millimeterbereich und befinden sich in unterschiedlicher Tiefe eines Objektes aus Glas, wie z.B. Rohglastafeln.

Rohglastafeln werden zu verschiedenen Endprodukten wie z.B. Isolierglas oder Sicherheitsglas für die Architektur weiterverarbeitet. Je nach Endprodukt können sich dann die Metallpartikel störend auf das Erscheinungsbild und die Funktionalität des Produktes auswirken. Aus diesem Grund ist es wichtig, bei der Glasverarbeitung möglichst die partikelhaltigen Bereiche des Glases herauszuschneiden und Glas mit geringer Partikelkonzentration oder partikelfreies Glas weiterzuverarbeiten.

### Stand der Technik

Im Stand der Technik sind optische Untersuchungsmethoden für einen Nachweis von Metallpartikeln in Flachglas bekannt, welche auf Reflexion und Streuung von Licht im Glas und Analyse des reflektierten und gestreuten Lichts basieren. Diese Methoden erlauben es, Einschlüsse und andere Glasfehler nachzuweisen. Beispiele für diese Detektionsmethoden finden sich in US 4,697,082, WO 01/73408 A1 oder WO 01/18532 A1.

Diese Methoden erfordern jedoch aufgrund der geringen Partikelgrößen eine hohe Ortsauflösung, sind daher aufwendig und erlauben es auch nicht, die Art der Metallpartikel hinsichtlich ihrer chemischen Zusammensetzung zu identifizieren. Letzteres kann jedoch für die Weiterverarbeitung des partikelhaltigen Rohglases von Bedeutung sein. Im Falle von z.B. Nickelsulfidpartikeln in Glas wäre eine Kenntnis des Partikeltypes von Vorteil. Wird dieses Glas z.B. zu Isolierglas weiterverarbeitet, so beeinträchtigen die Partikel nicht die Funktionalität des Glasproduktes. Lediglich wirken sich größere Partikel im Erscheinungsbild des Glasproduktes nachteilig aus. Bei der Herstellung von thermisch vorgespanntem Sicherheitsglas spielt das Vorhandensein von Nickelsulfid-einschlüssen im Glas für die Funktionalität des Sicherheitsglases eine entscheidende Rolle. Ein bekanntes Problem bei Glas, insbesondere bei grossflächigem Fassadenglas, besteht in dem spontanen Auftreten von Brüchen aufgrund von thermisch-induzierten Kristallumformungen von Nickelsulfidpartikeln, die zu zusätzlichen Spannungen im Glas und letzendlich zum Glasbruch führen. Aus diesem Grunde ist seit Beginn des Jahres 2003 vor dem Einbau von Fassadenglas ein sogenannter Heißlagerungstest (HST) gesetzlich vorgeschrieben, bei dem das Glas einer mehrstündigen Wärmebehandlung bei 290°C +-10°C unterworfen wird. Bei korrekter Durchführung dieses Tests ist das Risiko eines späteren Spontanbruchs wesentlich reduziert, aber es existiert noch. Könnte das Vorhandensein der Nickelpartikel vor dem Fertigungsprozeß des Sicherheitsglases identifiziert werden, würden so belastete Gläser gar nicht erst zu Sicherheitsglas verarbeitet. Damit wäre der Heißlagerungstest als Qualitätsprüfung überflüssig und durch Nickelsulfid verursachte Spontanbrüche würden nicht mehr auftreten. WO2006/074494 A1 offenbart ein Verfahren zur Prüfung auf Nickelsulfideinschlüsse in Sicherheitsglas wobei das Glas erwärmt wird und charakteristische Strahlung gemessen wird. Jedoch erlaubt dieses Verfahren keine Zuverlässige Identifikation und Unterscheidung verschiedenartiger Einschlüsse.

### Aufgabe der Erfindung

Demgemäß besteht die Aufgabe der vorliegenden Erfindung in der Bereitstellung eines zuverlässigen Verfahrens und einer entsprechender Vorrichtung zumindest zur vereinfachten Detektion und Identifizierung von Partikel, besonders von Metallpartikeln in Glas.

Diese Aufgabe wird mit einem Verfahren und einer Vorrichtung mit den Merkmalen der Hauptansprüche gelöst. Vorteilhafte Ausführungsformen der Erfindung sind Gegenstand der abhängigen Ansprüche.

Im Gegensatz zum obengenannten Stand der Technik beruht das erfindungsgemäßen Detektionsverfahren auf einer Erwärmung, insbesondere lokal begrenzten Erwärmung der Partikel durch Laserstrahlung, die das Glas zumindest im Wesentlichen absorptionsfrei durchdringt und im Vergleich zum Glas von eingeschlossenen Partikeln, insbesondere Metallpartikeln stärker absorbiert wird.

Die im Inneren des Glases lokal durchgeführte Erwärmung des Partikels führt gleichzeitig aufgrund von Wärmeleitungseffekten zu einer Erwärmung des das Partikel umgebenden Glases. Es wird daher durch diesen Wärmeleitungseffekt, insbesondere innhalb einer vorgegebenen Zeitdauer der Beleuchtung in einer dem Partikel nahen Glasumgebung eine stärkere Erwärmung gegenüber einer dem Partikel ferneren Glasumgebung stattfinden. Durch die Beleuchtungsdauer kann dabei auch Einfluß genommen werden auf die örtliche Größe der erwärmten, um ein Partikel angeordneten Glasumgebung. Z.B. kann eine Größe der erwärmten Glasumgebung um ein Partikel von größer 1 mm erreicht werden.

Hierdurch erschließt sich ein Vorteil gegenüber dem Stand der Technik, dass nämlich durch die Wärmeleitung ein größerer Bereich als das eigentliche Partikel erwärmt wird. Daher kommt das erfindungsgemäße Verfahren mit einer geringeren Ortsauflösung zurecht als bisherige Verfahren.

Diese somit lokal begrenzte Erwärmung kann mit wenigstens einer Wärmebildkamera, erfaßt werden. Somit wird die Lage des Partikels im Glas detektiert, z.B. durch Bildauswertung und Feststellung der örtlichen Lage einer Erwärmung im thermografischen Abbild der Wärmbildkamera. Hierfür muss das Abbild nicht visuell dargestellt werden, wenngleich dies möglich ist. Für die Feststellung der Lage und ggfs. einer weiteren Identifikation reicht es aus, das latente, d.h nicht visuell dargestellte und nur durch Messwerte repräsentierte thermografische Abbild auszuwerten.

Zusätzlich bietet das Verfahren auch eine Identifizierung der Art der Partikel hinsichtlich ihrer chemischen Zusammensetzung. Dazu wird die materialabhängige Erwärmung des Partikels hinsichtlich des Temperatur-Zeitverhaltens sowie die vom erwärmten Partikel emittierte materialabhängige, elektromagnetische Strahlung gemessen und ausgewertet. Damit ist eine Klassifizierung der Partikel hinsichtlich ihrer chemischen Zusammensetzung möglich.

Es wird hierfür ein festgestelltes Temperatur-Zeitverhalten, insbesondere das bei der Bestrahlung innerhalb der Bestrahlungszeit erzeugte Temperaturintervall zwischen anfänglicher Starttemperatur und am Ende der Bestrahlungsdauer erzeugter Endtemperatur oder aber auch eine erfasste zeitliche Abkühlkurve verglichen werden mit mehreren gespeicherten Temperatur-Zeitverhalten von bekannten Partikelarten.

Zusätzlich kann nach der Ermittlung von Lage und Typ des Partikels über eine z.B. zweite Laserbestrahlung das Partikel so stark erwärmt werden, daß die dadurch initierte thermische Ausdehnung zur Rißbildung der näheren Glasumgebung führt. Durch diese Rißbildung und der damit verbundenen Lichtstreuung wirkt das Partikel größer. Es wurde dekoriert. Damit kann es auch ohne Meßtechnik vom menschlichen Auge erfaßt werden und der Aussortierungsprozeß des belasteten Glases vereinfacht werden.

### Beispiel:

Eine Rohglastafel wird am sogenannten kalten Ende einer Flachglasherstellung auf einer Förderstrecke horizontal bis zum Abstapelvorgang bewegt. Während dieses Vorganges durchläuft die Tafel eine erfindungsgemäße Laserdetektions- und Identifizierungsvorrichtung, derart, daß Laserstrahlen mit z.B. linienförmigen, rechteckigem Strahlquerschnitt die Glastafel während ihrer Bewegung senkrecht durchleuchten. Gleichzeitig wird mit einem entsprechenden Detektionssystem oberhalb und/oder unterhalb der Glastafel das Wärmebild aufgenommen und entsprechende "Hot-Spots" (deutlich erwärmte Punkte im Vergleich zum kalten Glas) registriert und in bevorzugter Weiterbildung anhand ihres Erwärmungsverhaltens oder auch Abkühlungsverhaltens identifiziert.

## Patentansprüche

1. Verfahren zur Detektion von Partikeln in Glas, insbesondere von metallischen Partikeln in Glas, insbesondere in Flachglasscheiben, **dadurch gekennzeichnet, daß** das Glas zumindest in einem Bereich mit Laserstrahlung durchleuchtet wird, welche durch das Glas weniger absorbiert wird als durch im Glas vorhandene Partikel und hierdurch bei in dem durchleuchteten Bereich vorhandenen Partikeln eine Erwärmung zumindest des jeweiligen Partikels, insbesondere eine stärkere Erwärmung einer zu einem Partikel nahen Glasumgebung gegenüber einer zu einem Partikel ferneren Glasumgebung erzeugt wird und mittels wenigstens einer Wärmebildkamera, mit der elektromagnetische Wellen im Infrarotbereich erfasst werden, diese Erwärmung ortsaufgelöst erfaßt und aus der erfassten Erwärmung die Lage des jeweiligen Partikels im Glas bestimmt wird und wobei während und/oder nach der Durchleuchtung mit Laserlicht ortsaufgelöst die Temperaturänderung in dem durchleuchteten Bereich in Abhängigkeit von der Zeit erfasst wird und anhand der Erwärmung in ihrer örtlichen Lage festgestellte Partikel hinsichtlich der chemischen Zusammensetzung identifiziert werden in Abhängigkeit eines zumindest an diesem Ort gemessenen Temperatur-Zeitverhaltens, wofür ein festgestelltes Temperatur-Zeitverhalten mit mehreren gespeicherten Temperatur-Zeitverhalten von bekannten Partikelarten verglichen wird.

2. Verfahren nach Anspruch 1, **dadurch gekennzeichnet, dass** mit der Wärmebildkamera elektromagnetische Wellen, im Wellenlängenbereich von 0,7 bis 1000 Mikrometern, bevorzugt im Wellenlängenbereich von 3,5 bis 15 Mikrometern erfasst werden.

3. Verfahren nach einem der vorherigen Ansprüche, **dadurch gekennzeichnet, dass** mit der Wärmebildkamera wenigstens eine ortsaufgelöste thermografische zumindest latente Abbildung des beleuchteten Bereichs erstellt wird und aus in der Abbildung festgestellten Erwärmungen, insbesondere lokal begrenzten Erwärmungen auf die örtliche Lage eines Partikels innerhalb einer jeweiligen, insbesondere lokal begrenzten Erwärmung geschlossen wird.

4. Verfahren nach einem der vorherigen Ansprüche, **dadurch gekennzeichnet, dass** aufeinanderfolgend mehrere Bereiche, insbesondere aneinander angrenzende Bereiche des Glases durchleuchtet werden und die Erwärmung in jedem der Bereiche mittels Wärmebildkamera erfasst wird, insbesondere wobei die mehreren Bereiche durch eine Relativbewegung zwischen einem Objekt aus Glas und einem Laserstrahl durchleuchtet werden.

5. Verfahren nach Anspruch 4, **dadurch gekennzeichnet, dass** ein Objekt aus Glas, insbesondere eine Flachglasscheibe zur Beleuchtung aufeinanderfolgender Bereiche durch wenigstens einen das Objekt durchdringenden ortsfesten Laserstrahl, insbesondere wenigstens einen linienförmig aufgeweiteten Laserstrahl transportiert wird, insbesondere wobei der durch den wenigstens einen Laserstrahl durchleuchtete Bereich von wenigstens einer ortsfesten Wärmebildkamera erfasst wird.

6. Verfahren nach einem der vorherigen Ansprüche, **dadurch gekennzeichnet, daß** der wenigstens eine verwendete Laserstrahl einen linienförmigen, rechteckigen Strahlquerschnitt besitzt.

7. Verfahren nach einem der vorherigen Ansprüche, **dadurch gekennzeichnet, daß** am Ort eines festgestellten Partikels, insbesondere Metallpartikels eine Laserbestrahlung, insbesondere eine zweite Laserbestrahlung des Glases erfolgt, mit der eine thermische Behandlung, insbesondere Ausdehnung des Partikels und hierdurch eine Rißbildung im Glas am Ort des Partikels oder in der an das Partikel angrenzenden Glasumgebung erzeugt wird, insbesondere um den Ort eines Partikels im Glas für das menschliche Auge visuell wahrnehmbar zu markieren.

8. Vorrichtung zur Detektion von Partikeln in Glas, insbesondere von metallischen Partikeln in Glas, insbesondere in Flachglasscheiben, **dadurch gekennzeichnet, daß** sie eine Laserlichtquelle umfasst, mit der ein in der Vorrichtung angeordnetes oder durch die Vorrichtung hindurchführbares Objekt aus Glas zumindest bereichsweise durchleuchtbar ist, wobei die Wellenlänge der Laserlichtquelle derart ausgewählt ist, dass das Laserlicht durch das Glas weniger absorbiert wird als durch im Glas vorhandene Partikel, insbesondere eine Wellenlänge im Bereich von 250-1400 Nanometer ausgewählt ist und sie eine Wärmebildkamera umfasst, mit der eine aufgrund der Durchleuchtung hervorgerufene Erwärmung von in einem durchleuchteten Bereich vorhandenen Partikeln und/oder eine stärkere Erwärmung einer zu einem Partikel nahen Glasumgebung gegenüber einer zu einem Partikel ferneren Glasumgebung erfassbar ist und mittels der aus der erfassten Erwärmung zumindest die Lage des jeweiligen Partikels im Glas bestimmbar ist, wobei die Vorrichtung eingerichtet ist während und/oder nach der Durchleuchtung mit Laserlicht ortsaufgelöst die Temperaturänderung in dem durchleuchteten Bereich in Abhängigkeit von der Zeit zu erfassen und anhand der Erwärmung in ihrer örtlichen Lage festgestellte Partikel hinsichtlich der chemischen Zusammensetzung zu identifizieren in Abhängigkeit eines zumindest an diesem Ort gemessenen Temperatur-Zeitverhaltens, wofür ein festgestelltes Temperatur-Zeitverhalten mit mehreren gespeicherten Temperatur-Zeitverhalten von bekannten Partikelarten vergleichbar ist.

## Claims

1. Method for detecting particles in glass, in particular metallic particles in glass, in particular in flat glass panes, **characterized in that** laser radiation is shone through the glass at least in one region, said laser radiation being absorbed less by the glass than by particles present in the glass, and, should particles be present in the region through which the laser has shone, heating of at least the respective particle, in particular stronger heating of glass surroundings near a particle in relation to glass surroundings further away from a particle, is hereby produced and this heating is captured in spatially resolved fashion by means of at least one thermal imaging camera, which captures electromagnetic waves in the infrared range, and the position of the respective particle in the glass is determined from the captured heating and wherein, while the laser light is shone through and/or thereafter, the change in temperature is captured as a function of time in spatially resolved fashion in the region through which laser light has been shone and particles whose spatial position has been determined on the basis of the heating are identified in respect of their chemical composition depending on a temperature-time characteristic measured at least at this location, for the purposes of which a determined temperature-time characteristic is compared to a plurality of stored temperature-time characteristics of known particle types.

2. Method according to Claim 1, **characterized in that** the thermal imaging camera captures electromagnetic waves in the wavelength range from 0.7 to 1000 micrometres, preferably in the wavelength range from 3.5 to 15 micrometres.

3. Method according to either of the preceding claims, **characterized in that** the thermal imaging camera is used to create at least one spatially resolved thermographic, at least latent image of the illuminated region and the spatial position of a particle within a respective instance of heating, in particular a locally restricted instance of heating, is deduced from instances of heating determined in the image, in particular locally restricted instances of heating.

4. Method according to any one of the preceding claims, **characterized in that** a plurality of regions, in particular mutually adjoining regions, of the glass successively have light shone therethrough and the heating in each of the regions is captured by means of a thermal imaging camera, in particular wherein the plurality of regions have a light shone therethrough by way of a relative movement between an object made of glass and a laser beam.

5. Method according to Claim 4, **characterized in that,** for the purposes of illuminating successive regions, an object made of glass, in particular a flat glass pane, is transported through at least one stationary laser beam that passes through the object, in particular at least one laser beam that has been linearly expanded, in particular wherein the region through which the at least one laser beam has shone is captured by at least one stationary thermal imaging camera.

6. Method according to any one of the preceding claims, **characterized in that** the at least one employed laser beam has a linear, rectangular beam cross section.

7. Method according to any one of the preceding claims, **characterized in that** laser irradiation, in particular second laser irradiation, of the glass is implemented at the location of a determined particle, in particular metal particle, by means of which laser irradiation a thermal treatment is produced, in particular expansion of the particle, and, as a result thereof, a formation of a crack in the glass at the location of the particle or in the glass surroundings adjoining the particle is generated, in particular in order to mark the location of a particle in the glass in a manner that is visually perceivable by the human eye.

8. Apparatus for detecting particles in glass, in particular metallic particles in glass, in particular in flat glass panes, **characterized in that** said apparatus comprises a laser light source which can, at least in regions, shine through an object made of glass that is arranged in the apparatus or that can be guided through the apparatus, wherein the wavelength of the laser light source is selected in such a way that the laser light is absorbed less by the glass than by particles present in the glass, in particular a wavelength in the range of 250-1400 nanometres is selected, and said apparatus comprises a thermal imaging camera, by means of which heating, caused by the light shining therethrough, of particles present in a region through which light shines and/or stronger heating of glass surroundings close to a particle in relation to glass surroundings further away from the particle is rendered capturable and by means of which camera at least the position of the respective particle in the glass is determinable from the captured heating, wherein the apparatus is configured to capture, as a function of time, the change in temperature in the region through which light shines in a spatially resolved manner during and/or after shining laser light though said region and to identify particles, determined in respect of their spatial position, in respect of the chemical composition on the basis of the heating, depending on a temperature-time characteristic measured at least at this location, for the purposes of which a determined temperature-time characteristic is compared to a plurality of stored temperature-time characteristics of known particle types.

## Revendications

1. Procédé pour détecter des particules dans du verre, en particulier des particules métalliques dans du verre, en particulier dans des plaques de verre plat, **caractérisé en ce que** le verre est éclairé au moins dans une zone avec un rayonnement laser, lequel est moins absorbé par le verre que par des particules présentes dans le verre et ainsi, dans le cas de particules présentes dans la zone éclairée, un échauffement au moins de la particule respective, en particulier un échauffement plus important d'un environnement de verre proche d'une particule par rapport à un environnement de verre plus éloigné d'une particule, est généré et cet échauffement est détecté à résolution spatiale au moyen d'au moins une caméra thermique, avec laquelle des ondes électromagnétiques dans le domaine infrarouge sont détectées, et la position de la particule respective dans le verre est déterminée à partir de l'échauffement détecté et dans lequel pendant et/ou après l'éclairage avec la lumière laser, le changement de température est détecté à résolution spatiale dans la zone éclairée en fonction du temps et des particules dont la position locale est constatée à l'aide de l'échauffement sont identifiées en termes de composition chimique en fonction d'au moins un comportement température-temps mesuré à cet endroit, ce pour quoi un comportement température-temps constaté est comparé avec plusieurs comportements température-temps enregistrés de types de particule connus.

2. Procédé selon la revendication 1, **caractérisé en ce que** des ondes électromagnétiques, dans la gamme de longueurs d'ondes de 0,7 à 1000 micromètres, de préférence dans la gamme de longueurs d'ondes de 3,5 à 15 micromètres, sont détectées avec la caméra thermique.

3. Procédé selon l'une quelconque des revendications précédentes, **caractérisé en ce qu'**au moins une représentation thermographique à résolution spatiale au moins latente de la zone éclairée est établie avec la caméra thermique et il est conclu à la position locale d'une particule à l'intérieur d'un échauffement respectif, en particulier localement limité, à partir des échauffements constatés dans la représentation, en particulier des échauffements localement limités.

4. Procédé selon l'une quelconque des revendications précédentes, **caractérisé en ce que** plusieurs zones successives, en particulier des zones du verre adjacentes l'une à l'autre, sont éclairées et l'échauffement dans chacune des zones est détecté au moyen d'une caméra thermique, en particulier dans lequel lesdites plusieurs zones sont éclairées par un mouvement relatif entre un objet en verre et un rayon laser.

5. Procédé selon la revendication 4, **caractérisé en ce qu'**un objet en verre, en particulier une plaque de verre plat, est transporté pour l'éclairage de zones successives par au moins un rayon laser stationnaire traversant l'objet, en particulier au moins un rayon laser linéairement élargi, en particulier dans lequel la zone éclairée par ledit au moins un rayon laser est détectée par au moins une caméra thermique stationnaire.

6. Procédé selon l'une quelconque des revendications précédentes, **caractérisé en ce que** ledit au moins un rayon laser utilisé possède une section transversale de rayon rectangulaire, linéaire.

7. Procédé selon l'une quelconque des revendications précédentes, **caractérisé en ce qu'**une irradiation par laser, en particulier une deuxième irradiation par laser du verre, a lieu à l'endroit où une particule, en particulier particule métallique, a été constatée, avec laquelle un traitement thermique, en particulier une dilatation de la particule et ainsi une fissuration dans le verre à l'endroit de la particule ou dans l'environnement de verre adjacent à la particule, est généré, en particulier pour marquer de manière visuellement perceptible pour l'oeil humain l'endroit d'une particule dans le verre.

8. Dispositif pour détecter des particules dans du verre, en particulier des particules métalliques dans du verre, en particulier dans des plaques de verre plat, **caractérisé en ce qu'**il comprend une source de lumière laser, avec laquelle un objet en verre agencé dans le dispositif ou pouvant être guidé à travers le dispositif peut être éclairé au moins par endroits, dans lequel la longueur d'onde de la source de lumière laser est sélectionnée de sorte que la lumière laser soit moins absorbée par le verre que par des particules présentes dans le verre, en particulier une longueur d'onde dans la gamme de 250-1400nanomètres est sélectionnée et **en ce qu'**il comprend une caméra thermique, avec laquelle un échauffement provoqué en raison de l'éclairage de particules présentes dans une zone éclairée et/ou un échauffement plus important d'un environnement de verre proche d'une particule par rapport à un environnement de verre plus éloigné d'une particule peut être détecté et au moyen de laquelle au moins la position de la particule respective dans le verre peut être déterminée à partir de l'échauffement détecté, dans lequel le dispositif est conçu pour détecter, pendant et/ou après l'éclairage avec la lumière laser, à résolution spatiale le changement de température dans la zone éclairée en fonction du temps et identifier des particules dont la position locale est constatée à l'aide de l'échauffement en termes de composition chimique en fonction d'au moins un comportement température-temps mesuré à cet endroit, ce pour quoi un comportement température-temps constaté peut être comparé avec plusieurs comportements température-temps enregistrés de types de particule connus.
